# EUROPEAN PATENT APPLICATION

(11) **EP 0 637 450 A2**
(43) Date of publication of application: **08.02.1995**
(21) Application number: 93112761.7
(22) Date of filing: 09.08.1993
(51) Int. Cl.: A61K 38/18, A61K 38/39, A61K 38/48, A61K 38/55, A61K 31/73, A61K 31/557, A61K 31/56, A61K 31/375

(54) **Composition for revitalizing scar tissue**

(30) Priority: 04.08.1993 US 99241
(71) Applicant: COLLAGEN CORPORATION, Palo Alto, California 94303 (US)
(72) Inventor: Berg, Richard A., Los Altos, CA 94024 (US); Rhee, Woonza Min, Palo Alto, CA 94306 (US)
(74) Representative: Schwan, Gerhard, Dipl.-Ing.

(57) **Abstract**

In accordance with the present invention, a method is disclosed for the remediation of scar tissue in a human or an animal by introducing into the scar tissue or into tissue adjacent the scar tissue a remedial composition comprising a bioactive material selected from naturally occurring growth factors, synthetic growth factors, active peptide segments of naturally occurring and synthetic growth factors, and mixtures thereof. Typically the remedial composition includes a biodegradable or non-biodegradable support matrix material to provide for timed release of the bioactive material.

Preferably, the support matrix material is biodegradable and is selected from the group consisting of collagen, glycosaminoglycan, gelatin, albumin, hyaluronic acid, heparin, oxidized cellulose, dextran, polyglycolic acid, polylactic acid, polyanhydride and mixtures thereof.

Most scar tissue, particularly in human beings, is very dense and is difficult for the remedial composition described above to penetrate. To render the scar tissue less dense and to spatially expand the scar tissue fibrils, it is preferable to introduce into the scar tissue a softening, expanding composition as well as the remedial composition containing the bioactive material. The softening, expanding composition can be introduced simultaneously with introduction of the remedial composition; however, it is preferable that the softening, expanding composition be introduced prior to the remedial composition. A preferred softening, expanding composition includes at least one dried collagen-comprising polymer, at least one polymer hydrogel and a non-aqueous liquid carrier material.

## Description

### BACKGROUND OF THE INVENTION

### 1. Field of the Invention

The present invention pertains to a composition for altering scar tissue to render the tissue more elastic, expanded and softened. The invention is particularly effective in treating contour deformities, fibrous scars, adhesions, stress urinary incontinence, fecal incontinence, and localized muscular dysfunction, where normal tissue function is compromised by the presence of fibrotic connective tissue or scar tissue.

### 2. Description of the Background Art

The present invention relates to a composition useful in treatment of localized muscular dysfunction due to scarring following surgery, irradiation, laceration, burns or infections by introducing a controlled release, bioactive substance having tissue normalization activity into the scar tissue. Potential applications for this invention include, but are not limited to, treating contour deformities, surgical scars and adhesions, urinary incontinence, fecal incontinence, dermal scars, vocal cord injury, and esophageal injury.

The term "injury" as used herein means a wound resulting from surgery, irradiation, burns, laceration, exposure to chemicals, viral infection or bacterial infection. The term "scar tissue" means fibrotic or collagenous tissue formed during the healing of a wound or other morbid process, or otherwise damaged tissue. The term "controlled release matrix" means any composition which will allow the slow release of a bioactive substance which is coupled to, mixed or admixed therein. The matrix can be a solid composition, a porous material, or a semi-solid, gel or liquid suspension containing the bioactive substance. The term "implant" means introduction of the bioactive material/matrix by means of injection, surgery, trochars, catheters or any other means whereby the bioactive material is introduced into the scar tissue. The term "bioactive material" means any composition that will promote the vascularization and normalization of tissue function. Examples of bioactive materials include any growth factor or cytokine that will alter the metabolism, biosynthetic process, or growth or function of connective tissue, muscle tissue, or nerve tissue.

Scar tissue is fibrotic tissue composed mostly of disorganized collagen fibrils. It is tissue that is poorly vascularized, poorly innervated, and inelastic. When the normal cellular elements and extracellular matrix of damaged tissue are replaced by a distorted accumulation of scar tissue, the normal function of the tissue is lost. The formation of scar tissue can create localized muscular dysfunctions and inhibit a variety of physiological functions. Patients treated using surgery and/or irradiation frequently tend to develop scar tissue in the area of the treatment, sometimes leading to adhesions. The scar tissue which replaces normal tissue does not have the elasticity and other functional characteristics of the tissue native to the area of treatment. As a result, normal functionality of tissue in the treated area is affected. For example, male patients who have had prostectomies and female patients who have been treated surgically or with irradiation in periurethral tissue areas may develop incontinence. The scar tissue which replaces normal periurethral tissue reduces tissue elasticity and may cause incomplete urethral coaptation which can inhibit bladder control, resulting in incontinence.

U.S. Patent No. 3,949,073 to Daniels et al., issued April 6, 1976, describes a method for augmenting hard or soft connective tissue, such as skin, tendon, cartilage, bone or interstitium, in a living mammal. A proteolytic enzyme-solubilized, purified, native, polymerized collagen composition is implanted into the mammal at the augmentation site. The polymerized collagen remains at the site as a stable, non-reactive fibrous mass of tissue which is rapidly colonized by host cells and vascularized.

The method is said to be useful in treating a number of congenital anomalies, acquired defects or cosmetic defects. The acquired defects include depressed scars, subcutaneous atrophy, acne pitting of the face, linear scleroderma with subcutaneous atrophy, and unilateral vocal cord paralysis.

U.S. Patent No. 4,424,208 to Wallace et al., issued January 3, 1984, discloses an injectable implant material for soft tissue augmentation comprising a dispersion of particles of cross-linked atelopeptide collagen and reconstituted fibrous atelopeptide collagen in a physiological aqueous carrier. Implants of this material have improved persistence relative to previously-used collagen implant materials, that is, they are absorbed into fluid components by the body less rapidly. This is said to be particularly important when collagen is used as a pharmaceutical carrier, as a surgical prothesis (in sutures and would dressings, for example), and as an implant material.

Examples are cited for repair of acquired defects (post traumatic, post surgical, post infectious) such as depressed scars, subcutaneous atrophy, enophthalmos in the enucleated eye, acne pitting of the face, linear scleroderma with subcutaneous atrophy, saddlenose deformity, Romberg's disease and unilateral vocal cord paralysis.

U.S. Patent No. 4,582,640 to Smestad et al., issued April 15, 1986, describes a crosslinked atelopeptide collagen that is substantially free of residual crosslinking agent. This crosslinked collagen, when suspended in physiological saline at a concentration of 35 mg/ml, exhibits a shear viscosity whose log varies linearly with the log of the shear rate. This collagen is dispersed in an isotonic aqueous medium for use in soft tissue dermal augmentation and is injectable for the formation of implants that have excellent persistence.

This collagen implant material may be injected intradermally or subcutaneously to augment soft tissue, to repair or correct congenital anomalies, acquired defects or cosmetic defects. Examples of congenital anomalies which can be treated are said to be, for example, hemifacial microsomia, malar and zygomatic hypoplasia, unilateral mammary hypoplasia, pectus excavatum, pectoralis angenesis, and felopharyngeal incompetence secondary to cleft palate repair of submucous cleft palate (as a retropharyngeal implant). Examples of acquired defects (post-traumatic, post-surgical, post infectious) which can be treated include depressed scars, subcutaneous atrophy, keratotic lesions, enophthalmos in the enucleated eye, acne pitting of the face, linear scleroderma with subcutaneous atrophy, saddle-nose deformity, Romberg's disease and unilateral vocal cord paralysis.

U.S. Patent No. 4,837,285 to Berg et al., issued June 6, 1989, discloses a collagen matrix that is used to deliver growth factors, proteins, and other bioactive materials to wounds. The matrix can be delivered by injection.

U.S. Patent No. RE. 33,375 to Luck, issued October 9, 1990 (as a reissue of U.S. Patent No. 4,619,913, issued October 28, 1986), discloses a proteinaceous composition of collagen or fibrinogen containing a cytotoxic drug or a proliferation inhibitor. Luck is directed to a method of treating cellular disorders involving abnormal cellular growths by introducing the composition at the site of the disorder.

U.S. Patent No. 5,024,841 to Chu et al., issued June 18, 1991, discloses collagen implants that are useful as wound healing matrices. The implants comprise a fibrillar atelopeptide collagen matrix having a density of about 0.01 to about 0.3 g/cm₃, a thickness of about 1-20 mm, and pores at least 80% of which are at least 35 micrometers in diameter. The implants are capable of promoting connective tissue deposition, angiogenesis, reepithelialization, and fibroplasia. The wound healing matrix is also said to serve as an effective sustained delivery system for bioactive agents. In particular, the wound healing implants are said to serve as an effective sustained delivery vehicle for bioactive additives such as, for example, heparin or other glycosaminoglycans; extracellular matrix proteins; antibiotics; and growth factors, for example epidermal growth factor (EGF), platelet derived growth factor (PDGF), fibroblast growth factor (FGF), connective tissue activating peptides (CTAP), transforming growth factors (TGFs) and the like.

U.S. Patent No. 5,112,608, to Scott et al., issued May 12, 1992, describes using protease Nexin I for wound healing and discloses a method of delivering protease Nexin I using a collagen matrix. Protease Nexin I is a bioactive material that inhibits serine proteinases and which promotes collagen accumulation and may act by promoting angiogenesis.

Copending U.S. Patent Application Serial No. 07/843,124 of Jeffrey S. Freed, filed February 28, 1992, discloses the use of injectable biomaterials, including collagen-containing compositions, optionally in combination with growth factors, to repair and augment anal sphincters for the purpose of restoring competency to incompetent sphincter muscles. This treatment modality is claimed to be effective in the repair of sphincter muscles which have either been structurally damaged by obstetric injuries, complications of fistula or fissure surgery, traumatic injuries, cancer, or deteriorated due to age, congenital disorders, systemic and metabolic diseases, acquired neurological defects, or diseases of the colon and rectum.

Published European Patent Application No. 92111651.3 of Clarence C. Lee, published February 10, 1993, under publication number 0 526 756 A1, describes a composition which is said to be effective in revitalizing scar tissue by introducing a bioactive substance having angiogenic activity into the scar tissue. The composition is said to be effective in treating stress urinary incontinence or localized muscular dysfunction.

Published PCT Patent Application No. PCT/US92/09871 of Ruoslahti et al., published May 27, 1993 under publication number WO 93/09800, discloses a method of inhibiting an activity of a cell regulatory factor comprising contacting the cell regulatory factor with a purified polypeptide, wherein the polypeptide comprises the cell regulatory factor-binding domain of a protein, and wherein the protein is characterized by a leucine-rich repeat of about 24 amino acids. The invention relates to methods for the prevention or reduction of scarring by administering decorin or a functional equivalent of decorin to a wound.

The prior art discloses a wide variety of time-release compositions for the slow release of various biologically active compounds. These time-release compositions frequently include collagen, but also include the use of various other pharmaceutically acceptable biodegradable carriers such as gelatin, heparin, oxidized cellulose and dextran.

The use of an implant of a biologically active compound which is timed-released from a collagen matrix into a given tissue area to achieve cell recruitment of native cells, vascularization and the development of collagenous connective tissue within the implant is described by Bentz et al. in a 1987 article. In "Cartilage Induction and Differentiation: The Role of Bone Derived Cartilage Inducing Factor (CIF-A)", Development and Diseases of Cartilage and Bone Matrix, pp. 137-147, Alan R. Liss, Inc. (1987), Bentz et al. describe the use of cartilage-inducing factor (CIF-A) released from a collagen carrier. The CIF-A-collagen matrix was implanted subcutaneously in rats. Within 72 hours, a massive recruitment of activated fibroblasts and endothelial cells from underlying muscle resulted in a region of fibroplasia surrounding the implants. By day 10, collagen implants containing CIF-A were infiltrated by activated fibroblasts and endothelial cells which deposited an extensive vascular connective tissue matrix.

The use of heparin in fibrillar collagen suspensions to promote a dose-dependent increase in average fibril diameter was described by McPherson et al. in "The Effects of Heparin on the Physicochemical Properties of Reconstituted Collagen", Collagen Rel. Res., Vol. 1, 1988, pp.65-82. The addition of heparin to fibrillar collagen implants significantly influenced the biologic response to these implants in the rat subcutaneous model as well as the guinea pig dermal wound model. This was exemplified by enhanced fibroblast invasion in the former model and an increased degree of vascularization and incorporation of the implant into the granulation tissue in the latter model.

The above-cited prior art describes the use of time-release compositions to promote vascularization and the expansion and softening of acquired defects of tissues (post-traumatic, post surgical, and post-infection) in general. However, when the acquired defect is post-traumatic or post-surgical scar tissue, the vascularization and cell recruitment from adjacent tissues is more difficult, because the scar tissue is comprised substantially of collagen fibrils which are disorganized and densely packed. It would be highly desirable to be able to decrease the density of densely packed collagen and to increase the vascularization and recruitment of cells from adjacent tissue, so the scar tissue would be better expanded and softened and function in a manner which more closely approaches that of the surrounding, native tissue.

### SUMMARY OF THE INVENTION

In accordance with the present invention, a composition is disclosed for the remediation of scar tissue in a human or animal by introducing into the scar tissue or into tissue adjacent the scar tissue the remedial composition comprising a bioactive material selected from naturally occurring growth factors, synthetic growth factors, active peptide segments of natural and synthetic growth factors, and mixtures thereof.

A softening, expanding composition may be introduced into the scar tissue to facilitate the action of the above-referenced remedial composition. The softening, expanding composition can be introduced simultaneously with the remedial composition. However, preferaby, the softening, expanding composition is introduced into a human or an animal using a two step process comprising the steps of:
(a) introducing into the scar tissue a softening, expanding composition; and
(b) introducing into the scar tissue or into tissues adjacent the scar tissue, a remedial composition comprising a bioactive material selected from naturally occurring growth factors, synthetic growth factors, active peptide segments of naturally occurring and synthetic growth factors, and mixtures thereof.

When using the two step process, the scar tissue softening and expanding composition is selected from the group consisting of: a composition including at least one dried collagen-comprising polymeric hydrogel and a liquid carrier material; a composition including at least one glycosaminoglycan-comprising polymeric hydrogel and a liquid carrier material; a composition comprising at least one enzyme capable of degradation of connective tissue components; and mixtures thereof.

In a preferred embodiment of the above-described two step method, when the softening, expanding composition is the dried collagen-comprising polymeric hydrogel, the liquid carrier material is a non-aqueous solvent and the hydrogel material is expanded or swells when exposed to water-containing fluids supplied from surrounding tissue. The dense spacing of the scar tissue collagen fibrils is altered, as the fibrils are forced apart due to expansion of the collagen-comprising polymeric hydrogel.

In another embodiment of the above-described two step method, an enzyme or mixture of enzymes is introduced as part of the softening, expanding first composition, whereby the scar tissue is partially degraded, making it softer and more pliable prior to delivery of the biologically active material into the scar tissue or the tissue adjacent the scar tissue. It is further possible to utilize enzymes which are capable of degrading matrix components which are included in the softening, expanding composition, as a technique for breaking up the dense scar tissue in a timed-delivery manner from the matrix.

The kinds of enzymes which can be used to provide partial degradation of either the scar tissue or a matrix component of the softening, expanding composition depend upon the material to be partially degraded and will be tailored for the given application. Examples of enzymes for partial degradation of scar tissue include, but are not limited to, collagenases, gelatinases and stromelysin, commonly referred to as the family of matrix metalloproteinases. Interstitial collagenase, referred to as matrix metalloproteinase I, cleaves types I, II, and III collagen. 72 K gelatinase and 92 K gelatinase degrade type IV collagen, and stromelysin degrades proteoglycan core protein and further activates latent forms of the collagenases and gelatinases. Additionally plasmin, and plasminogen activator activate interstital collagenase and cathepsin G activates neutrophil collagenase.

Typically, the bioactive material is injected into the scar tissue or native tissue adjacent the scar tissue. Injection is preferred for introduction of the softening, expanding composition as well, particularly due to the density of the scar tissue which must be penetrated by this softening, expanding composition and to localize the activity of the softening, expanding composition to the target area.

Preferably, the bioactive material is used in combination with a matrix material which is selected from the group consisting of collagen, glycosaminoglycan, gelatin, albumin, hyaluronic acid, heparin, oxidized cellulose, dextran, polyglycolic acid, polylactic acid and polyanhydride, wherein the bioactive material is immobilized in the matrix and is present in an amount of about 0.001% to about 35 % by weight, based on the weight of the total mixture.

Preferably the softening, expanding composition is such that the solid or polymeric components, other than the non-aqueous solvent, are present in an amount of about 0.01 to about 25 % by weight based on the total weight of the mixture, whereby the softening, expanding composition can be injected through a conduit such as a needle.

Further in accordance with the present invention, a composition is described which is useful in scar tissue remediation in a human or animal. The remedial composition comprises a bioactive material selected from naturally occurring growth factors, synthetic growth factors, active peptide segments of naturally occurring or synthetic growth factors and mixtures thereof.

Preferred bioactive materials include, but are not limited to, growth factors selected from the group consisting of insulin-like growth factor, platelet-derived growth factor, transforming growth factor beta, and transforming growth factor alpha; connective tissue components such as fibronectin, type IV collagen, laminin, tenascin, and hyaluronic acid; drugs such as prostaglandins, retinols, retinoic acid, ascorbates, and estrogens; polysaccharides such as chitin or chitosan; enzyme inhibitors such as Protease Nexin I and Hirudin, other inhibitors of serine proteases and soluble receptors such as TGF-p.

The remedial composition typically additionally comprises a pharmaceutically acceptable controlled release matrix selected from collagen, gelatin, albumin, chondroitin sulfate, hyaluronic acid, heparin, oxidized cellulose, dextran, polyglycolic acid, polyactic acid and polyanhydride, wherein the bioactive material is immobilized in the matrix and is present in an amount of from about 0.001% to about 35 % by weight based on the total weight of bioactive material and matrix.

The remedial composition typically comprises a liquid carrier which makes it possible to inject the remedial composition through a conduit such as a needle. The liquid carrier is typically present in an amount ranging from about 75 to about 99.999 % by weight, based on the weight of the total composition including bioactive material, matrix material (when present) and liquid carrier. The liquid carrier is preferably selected from the group consisting of aqueous solution, mixtures of aqueous and organic solvents, and organic solvents such as glycols, nonionic dispersing agents, natural or synthetic oils, alcohols and combinations thereof.

An additional composition is disclosed which is useful in combination with the above-described remedial composition, which additional composition is a softening, expanding composition used to enable better penetration of the remedial composition into the scar tissue. The softening, expanding composition is selected from the group consisting of a composition comprising: at least one dried collagen-comprising polymeric hydrogel and a non-aqueous liquid carrier material; at least one glycosaminoglycan-comprising polymeric hydrogel and a non-aqueous liquid carrier material; a composition conprising at least one enzyme capable of degrading connective tissue components; and mixtures thereof. The softening, expanding composition is more effectively injectable when it is suspended in a non-aqueous solvent. Preferred non-aqueous solvents include liquids that do not promote swelling of the hydrogel. Such liquids include, but are not limited to, polyethylene glycol, polypropylene glycol, glycerol, fatty acids, vegetable oils such as soybean, peanut or sesame oil, lecithin, and long-chain alcohols having at least 3 carbon atoms. When the non-aqueous solvent diffuses from the dried collagen-comprising polymeric hydrogel and body fluids come into contact with the hydrogel, the hydrogel swells.

The remedial composition used alone or in combination with the softening, expanding composition can be used to treat the following tissues, by way of example, and not by way of limitation: urinary incontinance; fecal incontinance; impotence caused by accumulation of scar tissue; scarring of the vocal cords; keloids and hypertrophic scars; esophogeal reflux; and fibroid scar tissue present in soft tissue. Further, the remedial composition used alone or in combination with the softening, expanding composition can be used to anchor an implant material in place within native tissue. In this latter application, the implant comprises a matrix system which becomes anchored in place as the vascularization and cell importation from surrounding tissue occurs. This anchoring is preferable to that achieved by scar tissue, since the anchoring tissue is biocompatible and approaches the composition of native, surrounding tissue. For example, the compositions of the present invention are useful in anchoring foreign-body implants such as mammary implants, penile implants and artificial urinary sphincters which are supported by a matrix, and encapsulated implants that are surrounded by fibrous tissue. Further, the compositions can also be used to treat adhesions where surgery is not warranted.

### DETAILED DESCRIPTION OF THE PREFERRED EMBODIMENTS

In accordance with the present invention, a remedial composition is injected into or placed adjacent to scar tissue to induce normalization of the scar tissue, including vascularization and cell importation from adjacent tissues into the scar tissue. Innervation can be achieved by exposing the tissue to nerve growth factor or nerve growth promoting molecules such as laminin. The remedial composition comprises a bioactive material which is typically immobilized in a matrix for controlled release. Release of the bioactive material can be slow or rapid, depending upon the nature of the scar tissue. In cases wherein the scar tissue is particularly dense and inpenetrable, a softening, expanding composition may have to be used in combination with the remedial composition to enable adequate vascularization and cell importation. Eventually, the remedial composition and the scar tissue become vascularized, infiltrated by fibroblasts, and typically infiltrated by imported cells from adjacent native tissue, or the remedial composition is replaced by blood vessels or fibroblasts and imported cells. Muscle cells can be encouraged to migrate into the former scar tissue if a differentiation program is initiated. This process renders the scar tissue more elastic and more like the surrounding native tissue. Frequently the remedied scar tissue is sufficiently altered that the tissue composition becomes functionally similar to the surrounding native tissue, whereby normal bodily functions which depend upon that tissue can be resumed. For example, after a prostatectomy, urinary incontinence can be improved after muscle regrowth. In the case of urinary incontinence caused by periuretheral scar tissue, by repeated injection of specific bioactive materials, the scar tissue can be expanded to achieve urethral coaptation and relieve the incontinence. Alternatively, the bioactaive material can be incorporated into a delivery substance for slow release into the scar tissue, thereby reducing the required number of injections.

Examples of bioactive materials which can be used in the present invention include, but are not limited to: pituitary or synthetic growth hormones, growth factors, active fragments of growth factors, fibronectin, materials composed of a matrix and growth factors or active segments of growth factors, interleukins, and materials or synthetic compounds which promote the vascularization, infiltration of fibroblast cells, and importation of native cells into the scar tissue. Pituitary growth hormones, both natural or synthetic, have broad effects on various organs and tissues. Generally speaking, a growth factor has more specific biological effects on limited types of cells, tissues or organs which possess the corresponding cell surface receptor. For example, nerve growth factor induces the regeneration of peripheral nerve cells. Mixtures of different bioactive materials can be used in practicing the present invention.

Preferred examples of bioactive materials which can be used with the present invention include, but are not limited to, pituitary growth hormones (hGH and bGH) and various growth factors such as fibroblast growth factors (FGF), insulin-like growth factors (IGF), platelet-derived growth factors (PDGF), and transforming growth factors, including transforming growth factor alpha and beta. The bioactive materials which can be used with the present invention also include active peptide segments of various growth factors, e.g., binding site peptides (dormants) of growth factors which bind the receptors of fibroblast or other cells. Bioactive materials also include angiogenic synthetic peptides having biological activities similar to growth factors. Additionally, vitamin A, ascorbate, retinols, and complexes of copper ions chelated by peptides, polysaccharides or organic chelating agents, fibronectin, prostaglandin E, or other similarly effective compounds can also act as bioactive materials. One skilled in the art can select a suitable bioactive material appropriate for the particular application of the present invention.

Any of the above-mentioned bioactive materials can be used in combination with a support matrix which is preferably biodegradable. Suitable biodegradable matrices that can be used in the present invention include, but are not limited to, collagen, gelatin, albumin, glycosaminoglycans, hyaluronic acid, heparin, oxidized cellulose, dextran, polyglycolic acid, polyactic acid, polyanhydride, or the like, and mixtures thereof. Collagen and hyaluronic acid are two of the more preferred biodegradable matrix materials since they exhibit less antigenicity than many other proteins. Collagen has been used widely in human applications. Gelatin, a protein derived from denatured collagen is another preferred biodegradable matrix material, although it tends to be degraded more rapidly than collagen. Gelatin has also been used extensively in human therapeutic applications.

Bioactive materials can also be crosslinked to each other to serve as their own matrix for controlled release purposes. These substances can be used alone or in any combination of two or more.

The bioactive materials are immobilized in a matrix to allow sustained release and site-specific action. The bioactive materials may be either covalently bonded to the matrix or ionically bonded to the matrix. The matrix itself can be either a biodegradable polymer/biopolymer or a nondegradable polymer. The covalent bonding between bioactive material and matrix can be achieved using covalent crosslinkers which are at least bifunctional. Preferred bifunctional crosslinkers include glutaraldehyde, divinylsulfone, diisocyanate, cyanogen bromide-activated cellulose, and 1,1-carbonyl diimidazol-activated polyethylene glycol or polypropylene glycol and difunctional succinimidyl esters of glutaryl polyethylene glycol. Methods for covalently binding growth factors to collagen matrices using polyethylene glycol are disclosed in U.S. Patent No. 5,162,430. The covalent crosslinking bonds between the bioactive material and the matrix, or between bioactive molecules, can also be formed by UV or gamma irradiation; however, the resultant product is presently somewhat unpredictable. The bioactive materials can be immobilized by ionic coupling to a matrix or to themselves. The bioactive materials can also be encapsulated with or without covalent and/or ionic bonds. One example of this is the liposome-encapsulated insulin-like growth factor. Bioactive materials can also be crosslinked to each other, as previously described, for sustained release.

The preferred method of introducing the controlled release composition comprising matrices containing bioactive materials is by injection through a conduit such as a trochar, needle, or catheter. The controlled release composition is injected preferably at multiple sites until the scar tissue is uniformly infiltrated with the controlled release composition. Typically, the controlled release composition needs to be administered only once; however, in some cases a second or third administration may be necessary to achieve desired results. In many cases, when the scar tissue is particularly dense or when repeated administration does not seem to be having the desired effect, it is advisable to use the present composition for softening and expanding the scar tissue in combination with composition comprising the bioactive-material-containing matrix. Scar tissue can be treated using laparoscopic surgery wherein the tissue is imaged radiographically by negative contrast so it can be located for remote injection.

As a biodegradable matrix is degraded by the body, the bioactive components are gradually released. Simultaneously, angiogenesis, fibroblast infiltration, collagen production, proteoglycan production, native cell recruitment, and chemoattractant activity increase at the implant site.

Non-biodegradable matrices can also be used in the present invention. When a non-biodegradable matrix is used according to the present invention, the composition with the bioactive material distributed in the non-biodegradable matrix is introduced into the scar tissue. The bioactive material then diffuses out of the matrix, thereby achieving the vascularization, fibroblast formation and cell importation of adjacent native tissue cells, as previously described. The non-biodegradable carriers should be selected so that atoms or complexes from the non-biodegradable matrix do not migrate to other parts of the body where they cause interference with normal body functions. Non-biodegradable carriers include compounds such as polyethylene glycol diacrylate polymers, polyethylene and polycarbonates, among others.

The present invention is further illustrated by the following examples.

### EXAMPLES

### Example 1

### Preparation of a dried collagen polymeric hydrogel (Collagen/Hydrogel):

METHOD I (grinding collagen/hydrogel block): 14 ml of fibrillar collagen at 35 mg/ml was transferred into a 20 cc syringe. 0.16 g of disuccinimidyl glutaryl polyethylene glycol (3400 dalton) was dissolved in 2 ml PBS (phosphate buffered saline) and then withdrawn into a second syringe (20 cc volume). These two components were homogenized by syringe-to-syringe mixing for about 5 minutes (50 passes), and then allowed to crosslink at 37 degrees for about 16 hours. The formed gel of collagen/hydrogel was broken into small pieces by pushing back and forth between the two syringes. The broken gel pieces were dried under a vacuum at room temperature. The dried particulate was ground with a mortar and pestle to obtain a fine particulate mixture. This method produced irregular shaped and sized particles (0.5-2.5 mm diameter).

### METHOD II (droplet formation of collagen gel in a crosslinking solution):

Collagen/hydrogel was produced by delivering a collagen gel droplet into a solution of D-SGPEG (difunctional-succinimidyl glutarate polyethylene glycol) (12% w/v). Crosslinking occurred at the surface of the collagen droplet first, then the shape of the spherical droplet was maintained through continuous stirring for 2-3 hours. After the solution was filtered, the gel filtrate was washed with PBS and permitted to dry under a constant flow of air until it was completely dried. This method produced more uniform sized particles (200-600 micron diameter) compared with the grinding method.

### Example 2

### Purification of collagenase and stromelysin according to the procedure of Lark et al. (Connective Tissue Research 25: 49-65, 1990):

Stromelysin and collagenase were purified as pro-enzymes from human gingiva fibroblasts obtained from patients undergoing treatment for periodontal disease. Minced tissue was obtained and cultured in Dulbecco's minimal essential medium (DMEM) containing 25 mm Hepes, 10% fetal calf serum and 5% penicillin-streptomycin. At confluence, media was removed and the cells were incubated with DMEM containing 1nM human interleukin-1 for 7 days. The enzyme pro-stromelysin was assayed using a ³H-labeled transferrin after activation by p-amino phenylmercuric acetate (APMA), using a trichloroacetic acid precipitation assay. Collagenase was assayed after activation by APMA, using ¹⁴C-labeled bovine collagen as the substrate and a dioxane precipitation technique described by Lark et al. The enzymes were purified by concentrating culture medium and performing chromatography using a DEAE Sephacel ion exchange column to remove proteoglycan, followed by chromatography on carboxy methyl (CM) cellulose, to remove procollagenase. The procollagenase was eluted from the CM cellulose using 0.35 M NaCl. The material that did not bind to the CM cellulose was chromatographed on Green-A Dye matrix where prostromelysin bound to the column. Pro-stromelysin was eluted with 0.3M NaCl. The pro-stromelysin was chromatographed on an agarose gelatin column to remove any gelatinase activity that may have been present in the pro-stromelysin preparation.

The purified pro-stromelysin and pro-collagenase were activated with trypsin, followed by removing the trypsin with α1 anti-trypsin bound to agarose. The enzyme pro-stromelysin binds to the polycationic Green-A resin, while pro-collagenase binds to the polyanionic resin CM cellulose.

### Example 3

### Preparation of an injectable delivery system for degrading scar tissue; a methylated collagen is used as a support matrix, combined with human stromelysin as the active degredation material:

Collagen is partially methylated in acidic methanol under anhydrous conditions according to the method of Miyata (US Patent No. 4,164,559). To methylated collagen is added purified human stromelysin at a concentration of 1 mg stromelysin to 20 mg. methylated collagen in PBS. The resultant suspension is homogenized throughly using a syringe to syringe method so the enzyme is uniformly associated with the injectable collagen. The collagen is then injected from the syringe into the scar tissue to be degraded.

### Example 4

Preparation of an injectable delivery system for degrading scar tissue; hyaluronic acid is used as a support matrix, combined with human gingival collagenase as the active degredation material.

Hyaluronic acid obtained from Lifecore Corporation, Chaska, MI, is diluted to a concentration of 3% wt/vol. in PBS. Purified human gingival collagenase at a concentration of 1mg/10 ml of the hyaluronic acid solution is added to the solution of hyaluronic acid in a syringe. After syringe-to-syringe mixing, the collagenase preparation is injected into the scar tissue to be degraded.

### Example 5

Preparation of a rejuvenating mixture of growth factors for scar tissue normalization.

Platelets are removed from a patient using plateletpheresis and are sonicated in PBS at a concentration of 10⁷ cells per ml PBS. The platelet extract is mixed with an equal volume of fibrillar collagen, available from Collagen Corporation, Palo Alto, CA, at a concentration of 70 mg/ml in PBS. The platelet extract is homogenized with fibrillar collagen using the syringe to syringe method. The fibrillar collagen at a concentration of 35 mg/ml of platelet-containing extract is injected into the scar tissue that has been loosened by the prior injection of a dried collagen/hydrogel of the kind described in Example 1 or by a scar tissue degrading system of the kind described in Examples 3 and 4.

### Example 6

Preparation of an angiogenic injectable preparation for treating scar tissue.

One hundred µg of recombinant human bFGF (basic fibroblastic growth factor), available from Genzyme Corp. of Cambridge, MA, was homogenized with 30 mg/ml fibrillar collagen (Collagen Corporation) containing 0.3 mg/ml heparin in PBS using the syringe to syringe method, to produce an injectable collagen/heparin/bFGF mixture for injection into scar tissue. The scar tissue is loosened by prior treatment with collagenase and/or stromelysin according to Examples 3 and 4 above. The enzyme treatment is performed until the scar tissue is loosened; typically, loosening requires a treatment period of up to about one week with enzymes. Loosening of the scar tissue by injection with swellable hydrogel according to Example 1 requires a similar period of treatment.

The above-described preferred embodiments of the present invention are not intended to limit the scope of the present invention as demonstrated by the claims which follow, as one skilled in the art can, with minimal experimentation, extend the disclosed concepts of the invention to the claimed scope of the invention.

## Claims

1. A composition for scar tissue remediation in a human or animal, comprising a bioactive material selected from the group consisting of naturally occurring growth factors, synthetic growth factors, active peptide segments of naturally occurring and synthetic growth factors and mixtures thereof.

2. The composition of Claim 1, wherein said bioactive material is selected from the group consisting of: insulin-like growth factor, platelet-derived growth factor, transforming growth factor beta, transforming growth factor alpha; fibronectin; Type IV collagen; laminin, tenascin; hyaluronic acid; prostaglandin E; retinols, retinoic acid; ascorbates; estrogen; Protease Nexin I; hirudin; chitin; chitosan; and soluble receptor TGF-p.

3. The composition of Claim 1, wherein said bioactive material is a growth factor derived from a platelet extract wherein platelets are obtained from said human or animal using plateletpheresis.

4. The composition of Claim 1, wherein said bioactive material is a growth factor derived from a platelet extract wherein platelets are obtained from a human or an animal using plateletpheresis.

5. The composition of any one of the preceding Claims, wherein said remedial composition includes a pharmaceutically acceptable controlled release matrix selected from the group consisting of collagen, glycosaminoglycan, gelatin, albumin, hyaluronic acid, heparin, oxidized cellulose, dextran, polyglycolic acid, polylactic acid and polyanhydride.

6. The composition of Claim 5, wherein the concentration of said bioactive material ranges from about 0.001 % to about 35 % by weight based on the combined weight of said bioactive material and said matrix material.

7. A composition useful for softening and expanding scar tissue, comprising a dried collagen capable of expanding upon contact with bodily fluids, whereby fibrils of said scar tissue are separated spacially from each other.

8. The composition of any one of the preceding Claims, wherein a liquid carrier material is present at a concentration ranging from about 75 % to about 99.999 % by weight, based on the total weight of the composition.

9. A composition useful for softening and expanding scar tissue, selected from the group consisting of: a composition comprising at least one dried collagen-comprising polymeric hydrogel and a liquid carrier material; a composition comprising at least one glycosaminoglycan-comprising polymeric hydrogel and a liquid carrier material; a composition comprising at least one enzyme capable of degradation of connective tissue components; and mixutures thereof.

10. The composition of Claim 8, wherein said liquid carrier is present at a concentration ranging from about 75 % to about 99.999 % by weight of the total composition.

11. The composition of any one of Claims 8 to 10, wherein said liquid carrier material is selected from the group consisting of glycerol, lecithin, vegetable oils, fatty acids, and long chain alcohols having at least 3 carbon atoms.

12. A composition of matter which is useful in scar tissue remediation in a human or animal, said composition comprising:
(a) a bioactive material selected from naturally occurring growth factors, synthetic growth factors, active peptide segments of naturally occurring and synthetic growth factors and mixtures thereof, and
(b) a dried collagen-comprising polymeric hydrogel capable of expanding upon contact with bodily fluids, whereby fibrils of said scar tissue are separated spacially from each other.
